# EUROPÄISCHE PATENTSCHRIFT

(11) **EP 3 184 754 B1**
(45) Veröffentlichungstag und Bekanntmachung des Hinweises auf die Patenterteilung: **08.07.2020**
(21) Anmeldenummer: 16205345.8
(22) Anmeldetag: 20.12.2016
(51) Int. Cl.: F01D 11/00, F01D 17/08, F01D 21/00

(54) **SENSORANORDNUNG UND MESSVERFAHREN FÜR EINE TURBOMASCHINE**
SENSOR ASSEMBLY AND MEASURING METHOD FOR A TURBOENGINE
SYSTÈME DE DÉTECTION ET PROCÉDÉ DE MESURE D'UNE TURBOMACHINE

(30) Priorität: 24.12.2015 DE 102015226732
(43) Veröffentlichungstag der Anmeldung: 28.06.2017
(73) Patentinhaber: Rolls-Royce Deutschland Ltd & Co KG, 15827 Blankenfelde-Mahlow (DE)
(72) Erfinder: FECHNER, Stefan, 15827 Blankenfelde-Mahlow (DE); ALBELT, Stefan, 15827 Blankenfelde-Mahlow (DE)
(74) Vertreter: Maikowski & Ninnemann Patentanwälte Partnerschaft mbB

(56) Entgegenhaltungen:
- EP-A1- 0 493 111
- DE-A1-102013 220 455
- GB-A- 2 062 763
- US-A1- 2013 272 860

## Beschreibung

Die Erfindung bezieht sich auf eine Sensorvorrichtung für eine Turbomaschine mit den Merkmalen des Anspruchs 1 und ein Messverfahren mit den Merkmalen des Anspruchs 6.

In Turbomaschinen, insbesondere Flugzeugtriebwerken, spielen Fluidströme an vielen Stellen eine Rolle. So ist es z.B. an verschiedenen Stellen der Turbomaschine notwendig, Kühlluft einzusetzen und deren Temperatur zu überwachen. Ein plötzlicher Temperaturanstieg der Kühlluft kann auf ein Problem hindeuten oder ein Problem verursachen. Auch kann es notwendig sein, Arbeitsfluide, wie z.B. Ölflüsse, zu überwachen.

In der EP 0 493 111 A1 ist eine Labyrinthdichtung in einer Gasturbine beschrieben. Ein Gasstrom bildet eine ringförmige Strömung aus und eine Sensorvorrichtung misst eine Temperatur an einem Zwischenpunkt des Strompfades.

Es besteht daher die Aufgabe, Sensoranordnungen und Messverfahren zu schaffen, die eine effiziente und zuverlässige Überwachung von Fluiden in der Turbomaschine ermöglichen.

Die Aufgabe wird auch durch eine Sensoranordnung mit den Merkmalen des Anspruchs 1 gelöst.

Dabei erfasst das Sensorelement mindestens eine Fluideigenschaft in einer berührungsfreien Dichtung zwischen einer Rotorstufe und einer Statorstufe einer Turbine, insbesondere einer Hochdruckturbine, wobei das Sensorelement im Betrieb einen Kontakt (direkt oder indirekt) mit der Fluidströmung entlang des Strömungspfades in der berührungsfreien Dichtung aufweist. Dabei ist das Sensorelement am Anfang, in der Mitte oder am Ende des Strömungspfades durch die berührungsfreie Dichtung angeordnet. Somit erfolgt die Erfassung direkt in der Dichtung und nicht an einer vorgelagerten oder nachgelagerten Stelle. Die Dichtung mit dem Sensorelement liegt in der Nähe des Gaspfades, um gegen Heißgaseintritt abzudichten und gegebenenfalls einen Heißgaseintritt zu detektieren.

In einer Ausführungsform besteht über den Strömungspfad ein Druckgefälle, so dass ein Fluid durch die berührungsfreie Dichtung, insbesondere die Labyrinthdichtung, transportiert wird.

Das Sensorelement kann zusätzlich auch in einer Messkammer positioniert sein, die durch einen Kanal mit der eigentlichen Messstelle in der Dichtung verbunden ist, und somit das zu detektierende Fluid erst durch einen Kanal zum Sensorelement geführt werden muss.

In Anwendung der Sensoranordnung kann das Sensorelement eine Temperatur der Fluidströmung, insbesondere einer Luftströmung, einen Mengenstrom der Fluidströmung, insbesondere einen Ölfluss und / oder eine Zusammensetzung der Fluidströmung, insbesondere ein Verbrennungsgas, messen. Dabei können z.B. optische Sensoren eingesetzt werden, die die Zusammensetzung, die Leitfähigkeit oder die optische Durchlässigkeit des Fluidstroms erfassen können.

Dabei ist es auch möglich, dass das Sensorelement mit einer Steuerungsvorrichtung gekoppelt ist, mit der in Abhängigkeit von der Messung des Sensorelementes ein Steuersignal abgebbar ist, welches beispielsweise den Piloten im Cockpit über die Messung informieren oder eine automatische Abschaltung des Flugzeugtriebwerkes einleiten kann.

Die Aufgabe wird auch durch ein Messverfahren mit den Merkmalen des Anspruchs 6 gelöst.

Ausführungsformen der Erfindung werden anhand der folgenden Figuren beispielhaft dargestellt. Es zeigt:
- Fig. 1: eine schematische Darstellung eines Flugzeugtriebwerkes;
- Fig. 2: eine schematische, vereinfachte Schnittansicht eines ersten Ausführungsbeispiels der erfindungsgemäßen Sensoranordnung in einer Axialschnittebene;
- Fig. 3: eine schematische, vereinfachte Schnittansicht eines zweiten Ausführungsbeispiels der erfindungsgemäßen Sensoranordnung in einer Axialschnittebene;
- Fig. 4: eine schematische Ansicht eines Fehlerfalls beim Ansaugen heißen Gases durch eine Labyrinthdichtung;
- Fig. 5: eine schematische Ansicht eines Fehlerfalls bei einem Ölleck in einer berührungsfreien Dichtung;
- Fig. 6: eine schematische Ansicht eines Fehlerfalls bei einer Verbrennung;
- Fig. 7: eine perspektivische Darstellung einer realen, berührungsfreien Dichtung mit einer Ausführungsform einer Sensoranordnung.

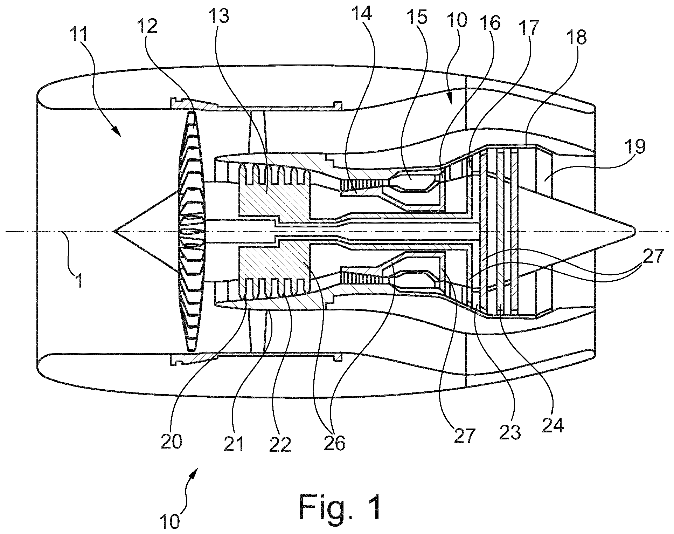

Das Flugzeugtriebwerk 10 gemäß Fig. 1 zeigt ein allgemeines Beispiel einer Turbomaschine.

Das Flugzeugtriebwerk 10 ist in an sich bekannter Weise meistens als Mehr-Wellen-Triebwerk ausgebildet und umfasst in Strömungsrichtung hintereinander einen Lufteinlass 11, einen in einem Gehäuse umlaufenden Fan 12, gegebenenfalls einen Mitteldruckkompressor 13, einen Hochdruckkompressor 14, eine Brennkammer 15, eine Hochdruckturbine 16, gegebenenfalls eine Mitteldruckturbine 17 und eine Niederdruckturbine 18 sowie eine Abgasdüse 19, die sämtlich um eine zentrale Triebwerksachse 1 angeordnet sind.

Der Mitteldruckkompressor 13 und der Hochdruckkompressor 14 umfassen jewells mehrere Stufen, von denen jede eine in Umfangsrichtung verlaufende Anordnung fester stationärer Leitschaufeln 20 aufweist, die allgemein als Statorschaufeln bezeichnet werden und die radial nach innen vom Triebwerksgehäuse 21 in einen ringförmigen Strömungskanal durch die Kompressoren 13, 14 vorstehen. Die Kompressoren weisen weiter eine Anordnung von Kompressorlaufschaufeln 22 auf, die radial nach außen von einer drehbaren Trommel oder Scheibe 26 vorstehen, die mit Turbinenrotornaben 27 der Hochdruckturbine 16 bzw. der Mitteldruckturbine 17 gekoppelt sind.

Die Turbinen 16, 17, 18 weisen ähnliche Stufen auf, umfassend eine Anordnung von festen Leitschaufeln 23, die radial nach innen vom Gehäuse 21 in den ringförmigen Strömungskanal durch die Turbinen 16, 17, 18 vorstehen, und eine nachfolgende Anordnung von Turbinenschaufeln 24, die nach außen von einer drehbaren Turbinenrotornabe 27 vorstehen. Die Kompressortrommel oder Kompressorscheibe 26 und die darauf angeordneten Schaufeln 22 sowie die Turbinenrotornabe 27 und die darauf angeordneten Turbinenlaufschaufeln 24 drehen sich im Betrieb um die Triebwerksachse 1.

Zwischen den Statoren und Rotoren der Kompressoren 13, 14 und Turbinen 16, 17, 18 befindet sich häufig ein Spalt, direkt am Strömungskanal aber auch weiter innerhalb des Triebwerkes, durch den Fluide, wie z.B. Kühlluft oder Öl, hindurchtreten können. Diese Spalte sind in der Regel mit berührungsfreien Dichtungen 40, wie z.B. einer Labyrinthdichtung, versehen. Dabei ist es für die Funktion des Flugzeugtriebwerks 10 wichtig, den Fluidfluss über die Dichtung 40 zu überwachen.

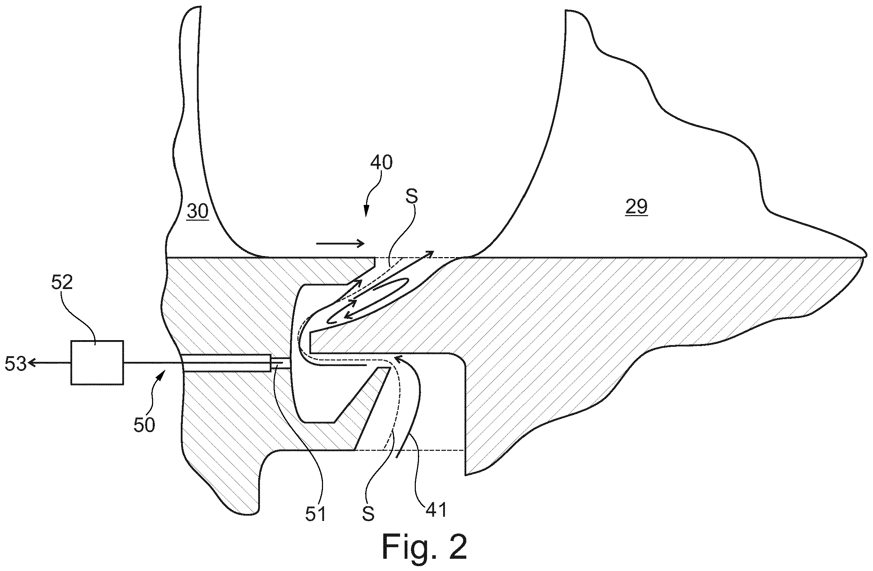

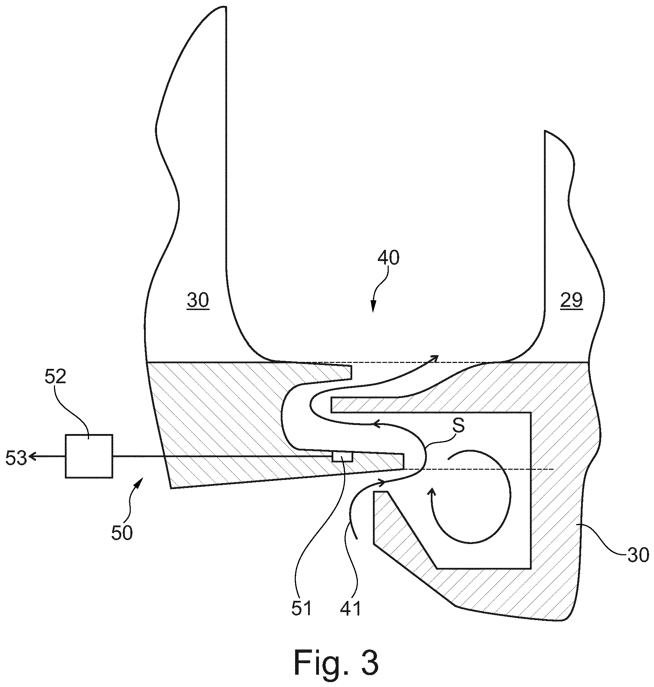

In Fig. 2 und 3 sind Ausführungsformen einer Labyrinthdichtung 40 mit einer Sensoranordnung 50 zur Fluidüberwachung dargestellt. Bei einer Labyrinthdichtung 40 beruht die Dichtwirkung auf einer Verlängerung des Strömungsweges im abzudichtenden Spalt, wodurch der Strömungswiderstand erhöht wird. Typischerweise weist eine Labyrinthdichtung 40 ineinandergreifende Teile auf (Verkämmung).

Die Schnittebenen der Fig. 2 und 3 umfassen die Triebwerksachse 1 und erstrecken sich somit in radialer Richtung.

Die Fig. 2 und 3 zeigen jeweils ein Ausführungsbeispiel, bei welchem die Strömungsrichtung im Flugzeugtriebwerk 10 von links nach rechts verläuft. Die Turbinenströmung durchströmt somit zunächst eine Statorstufe 30 mit Leitschaufelblättern, bevor sie auf eine Rotorstufe 29 mit Laufschaufelblättern auftrifft.

In Fig. 2 ist die Labyrinthdichtung 40 zwischen der Statorscheibe 30 und der Rotorscheibe 29 angeordnet. Die Fluidströmung 41 - hier z.B. Luft - strömt radial von innen nach außen entlang dem Strömungspfad S. Der Strömungspfad wird hier radial innen und außen durch die gestrichelte Linie begrenzt.

Direkt am Strömungspfad S ist hier ein Sensorelement 51 als Teil einer Sensorvorrichtung 50 angeordnet, mit der hier eine Fluideigenschaft, nämlich die Temperatur in der Labyrinthdichtung 40, gemessen wird. Dabei hat das Sensorelement 51 Kontakt (direkt oder indirekt) mit der Fluidströmung 41 entlang des Strömungspfades S in der Labyrinthdichtung 40. Das Sensorelement 41 ist hier in etwa der Mitte des Strömungspfades S angeordnet und so orientiert, dass die Fluidströmung 41 auf das Sensorelement 41 zuströmt.

In Fig. 3 ist eine alternative Sensoranordnung 50 in einer Labyrinthdichtung 40 dargestellt, wobei auf die Beschreibung der Fig. 2 Bezug genommen werden kann. Auch hier ist das Sensorelement 51 in etwa in der Mitte des Strömungspfades S angeordnet. Allerdings wird das Sensorelement 51 hier tangential angeströmt.

In alternativen Ausführungsformen ist das Sensorelement 41 am Anfang oder Ende des Strömungspfades S angeordnet. Auch ist es möglich, dass das Sensorelement 51 nicht im rechten Winkel zum Strömungspfad S, sondern in einem spitzen oder stumpfen Winkel angeordnet ist. In jedem Fall hat es direkten Kontakt mit dem in der Labyrinthdichtung 40 fließenden Fluid.

In den Ausführungsformen der Fig. 2 und 3 ist das Sensorelement 51 der Sensoranordnung 50 mit einer Steuervorrichtung 52 verbunden, die ein Steuersignal 53 abgeben kann, wenn z.B. ein bestimmter Messwert - hier ein Temperaturwert - über- oder unterschritten wird.

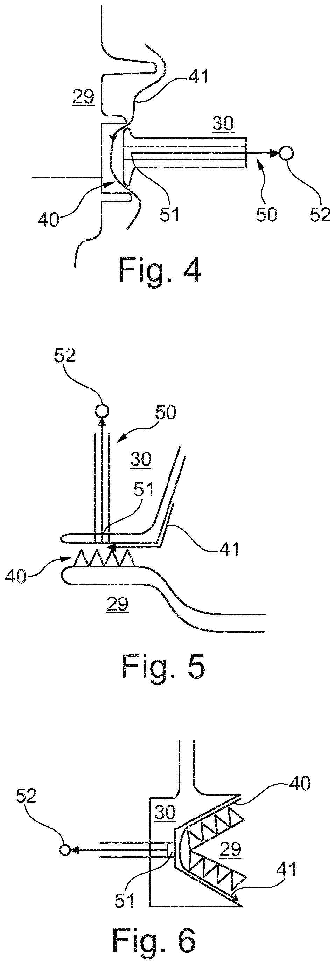

Wenn z.B. auf Grund einer Fehlfunktion heißes Gas von außen nach innen durch die Labyrinthdichtung 40 strömen würde, so würde der Temperaturanstieg detektiert werden. Dies könnte dann über die Steuervorrichtung 52 in ein Steuersignal 53 umgesetzt werden. In Fig. 4 bis 6 sind einige solche Fälle schematisch dargestellt. In allen drei Fällen ist das Sensorelement 51 in etwa in der Mitte des Fluidflusses 41 angeordnet. Die Sensoranordnung ist jeweils in der Statorstufe 30 angeordnet.

In Fig. 4 ist eine Labyrinthdichtung 40 dargestellt, die zwischen einer Rotorstufe 29 links und einer Statorstufe 30 rechts angeordnet ist. Hier ist der Fall dargestellt, dass bei einem Schadensfall im Flugzeugtriebwerk 10 heißes Gas von außen radial nach innen durch die Labyrinthdichtung 40 strömt. Das Sensorelement 51 detektiert den Temperaturanstieg, der ein Indikator für den Schaden ist und leitet die Information an die Steuervorrichtung 52 weiter. Bei der Überschreitung eines definierten Schwellenwertes könnte der Pilot im Cockpit über ein Signal informiert werden. Des Weiteren wäre eine automatische Abschaltung des Flugzeugtriebwerkes möglich.

In Fig. 5 ist eine weitere Anwendung der Sensoranordnung 50 dargestellt, bei der es um die Erfassung eines Öllecks geht. Hier ist eine berührungslose Dichtung 40 zwischen einer Statorstufe 30 oben und einer darunterliegenden Rotorstufe 29 angeordnet. Ein Sensorelement 51, z.B. eines optischen Ölsensors, erfasst z.B. ein Leck einer Lagerkammer, wenn der Ölfluss über eine bestimmte Menge hinausgeht. Das Messergebnis wird an die Steuervorrichtung 52 weitergeleitet.

In Fig. 6 ist eine Labyrinthdichtung 40 zwischen einer Statorstufe 30 links und einer Rotorstufe 29 rechts angeordnet. Hier erfasst ein optischer Sensor, ob Verbrennungsprodukte und / oder Festkörper mit dem Fluid durch die Labyrinthdichtung 40 strömen. In diesem Fall würden sich die optischen Eigenschaften (z.B. Extinktion) des Fluids verändern, so dass ein Fehlerfall des Eintritts von Verbrennungsgasen über das Dichtungselement erfasst werden könnte. Auch hier wird das Messergebnis an die Steuervorrichtung 52 weitergeleitet.

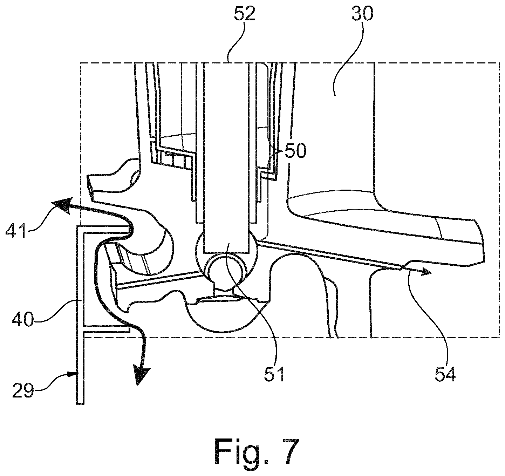

In Fig. 7 ist eine perspektivische Ansicht einer berührungsfreien Dichtung 40 zwischen einer Statorstufe und einer Rotorstufe einer Turbine dargestellt. In Fig. 7 ist die Fluidströmung 41 dargestellt, die sich z.B. auf Grund eines ungeplanten Ansaugens eines heißen Gases einstellt. Das Sensorelement 51 wirkt in diesem Fall nicht direkt am Ort der Fluidströmung 41. Damit das heiße Gas jedoch ebenfalls unmittelbar detektiert werden kann, bevor es weiter ins Innere des Flugzeugtriebwerks 10 eintritt, wird die Fluidströmung 41 durch ein über der Statorstufe vorhandenes Druckgefälle abgesaugt und über einen Luftkanal 54 an dem Sensorelement vorbeigeführt. Durch das Auslegen eines kurzen Kanals wird eine verzögerte Reaktion auf den Eintritt des heißen Gases minimiert, jedoch auch eine vereinfachte Ausführung, hier insbesondere eines geraden Sensorelementes 51, ermöglicht. Diese einfache Bauweise des Sensors ermöglicht dann einen Austausch des Elementes mit Triebwerken, welche noch am Flugzeug montiert sind.

In der hier dargestellten Ausführungsform weist die Sensoranordnung 50 ein in etwa zylindrisches Sensorelement 51 auf.

### Bezugszeichenliste

- 1: Triebwerksachse
- 10: Gasturbinentriebwerk, Flugzeugtriebwerk
- 11: Lufteinlass
- 12: Fan
- 13: Mitteldruckkompressor (Verdichter)
- 14: Hochdruckkompressor
- 15: Brennkammer
- 16: Hochdruckturbine
- 17: Mitteldruckturbine
- 18: Niederdruckturbine
- 19: Abgasdüse
- 20: Kompressorleitschaufeln
- 21: Triebwerksgehäuse
- 22: Kompressorlaufschaufeln
- 23: Turbinenleitschaufeln
- 24: Turbinenlaufschaufeln
- 26: Kompressortrommel oder-scheibe
- 27: Turbinenrotornabe

- 29: Rotorstufe
- 30: Statorstufe

- 40: Dichtung, Labyrinthdichtung
- 41: Fluidströmung

- 50: Sensoranordnung
- 51: Sensorelement
- 52: Steuerungsvorrichtung
- 53: Steuersignal
- 54: Luftkanal

- S: Strömungspfad in Labyrinthdichtung

## Patentansprüche

1. Sensoranordnung (50) mit einem Sensorelement (51) für die Messung mindestens einer physikalischen und / oder chemischen Fluideigenschaft in einer Turbomaschine (10), die eine Turbine (16, 17, 18), insbesondere einer Hochdruckturbine (16), aufweist, in der eine Rotorstufe (29) und eine Statorstufe (30) angeordnet sind,
**dadurch gekennzeichnet, dass**
das Sensorelement (51) die mindestens eine Fluideigenschaft in einer berührungsfreien Dichtung, insbesondere einer Labyrinthdichtung (40), zwischen der Rotorstufe (29) und der Statorstufe (30) erfasst, wobei das Sensorelement (51) im Betrieb einen Kontakt mit der Fluidströmung (41) entlang des Strömungspfades (S) in der berührungsfreien Dichtung (40) aufweist, wobei das Sensorelement (51) am Anfang, in der Mitte oder am Ende des Strömungspfades (S) durch die berührungsfreie Dichtung angeordnet ist, wobei die Dichtung (40) mit dem Sensorelement (51) in der Nähe des Gaspfades liegt, um gegen Heißgaseintritt abzudichten und gegebenenfalls einen Heißgaseintritt zu detektieren.

2. Sensoranordnung nach Anspruch 1, **dadurch gekennzeichnet, dass** über den Strömungspfad (S) ein Druckgefälle besteht, so dass ein Fluid durch die berührungsfreie Dichtung, insbesondere die Labyrinthdichtung (40), transportiert wird.

3. Sensoranordnung nach mindestens einem der vorhergegangen Ansprüche, **dadurch gekennzeichnet, dass** das Sensorelement (51) in einer Messkammer positioniert ist, die durch einen Kanal mit der eigentlichen Messstelle in der Dichtung (40) verbunden ist, und somit das zu detektierende Fluid erst durch einen Kanal zum Sensorelement (51) geführt werden muss.

4. Sensoranordnung nach mindestens einem der vorhergehenden Ansprüche, **dadurch gekennzeichnet, dass** mit dem Sensorelement (51) eine Temperatur der Fluidströmung (41), insbesondere einer Luftströmung, ein Mengenstrom der Fluidströmung (41), insbesondere ein Ölfluss und / oder eine Zusammensetzung der Fluidströmung (41), insbesondere eines Verbrennungsgases, messbar ist.

5. Sensoranordnung nach mindestens einem der vorhergehenden Ansprüche, **dadurch gekennzeichnet, dass** das Sensorelement (51) mit einer Steuerungsvorrichtung (52) gekoppelt ist, mit der in Abhängigkeit von der Messung mit dem Sensorelement (51) ein Steuersignal (53) abgebbar ist.

6. Messverfahren mit einem Sensorelement (51) für die Messung mindestens einer physikalischen und / oder chemischen Fluideigenschaft in einer Turbomaschine (10), die eine Turbine (16, 17, 18), insbesondere einer Hochdruckturbine (16), aufweist, in der eine Rotorstufe (29) und eine Statorstufe (30) angeordnet sind,
**dadurch gekennzeichnet, dass**
das Sensorelement (51) die mindestens eine Fluideigenschaft in einer berührungsfreien Dichtung, insbesondere einer Labyrinthdichtung (40), zwischen der Rotorstufe (29) und der Statorstufe (30) erfasst, wobei die Messung durch das Sensorelement (51) im Betrieb durch einen Kontakt mit der Strömung des Fluides entlang des Strömungspfades (S) in der berührungsfreien Dichtung (40) erfolgt, wobei das Sensorelement (51) am Anfang, in der Mitte oder am Ende des Strömungspfades (S) durch die berührungsfreie Dichtung angeordnet ist, wobei die Dichtung (40) mit dem Sensorelement (51) in der Nähe des Gaspfades liegt, um gegen Heißgaseintritt abzudichten und gegebenenfalls einen Heißgaseintritt zu detektieren.

## Claims

1. Sensor arrangement (50) having a sensor element (51) for measuring at least one physical and/or chemical fluid property in a turbomachine (10) having a turbine (16, 17, 18), in particular a high-pressure turbine (16), in which a rotor stage (29) and a stator stage (30) are arranged,
**characterized in that**
the sensor element (51) captures the at least one fluid property in a non-contact seal, in particular a labyrinth seal (40), between the rotor stage (29) and the stator stage (30), wherein the sensor element (51) is in contact with the fluid flow (41) along the flow path (S) in the non-contact seal (40) during operation, wherein the sensor element (51) is arranged at the start, in the middle or at the end of the flow path (S) through the non-contact seal, wherein the seal (40) with the sensor element (51) is in the vicinity of the gas path in order to provide sealing against the ingress of hot gas and possibly to detect an ingress of hot gas.

2. Sensor arrangement according to Claim 1, **characterized in that** there is a pressure drop across the flow path (S), with the result that a fluid is transported through the non-contact seal, in particular the labyrinth seal (40).

3. Sensor arrangement according to at least one of the preceding claims, **characterized in that** the sensor element (51) is positioned in a measurement chamber which is connected to the actual measurement point in the seal (40) by means of a channel, and the fluid to be detected must therefore first be conducted through a channel to the sensor element (51) .

4. Sensor arrangement according to at least one of the preceding claims, **characterized in that** the sensor element (51) can be used to measure a temperature of the fluid flow (41), in particular an air flow, a volume flow of the fluid flow (41), in particular an oil flow, and/or a composition of the fluid flow (41), in particular a combustion gas.

5. Sensor arrangement according to at least one of the preceding claims, **characterized in that** the sensor element (51) is coupled to a control apparatus (52) which can be used to emit a control signal (53) on the basis of the measurement with the sensor element (51) .

6. Measurement method with a sensor element (51) for measuring at least one physical and/or chemical fluid property in a turbomachine (10) having a turbine (16, 17, 18), in particular a high-pressure turbine (16), in which a rotor stage (29) and a stator stage (30) are arranged,
**characterized in that**
the sensor element (51) captures the at least one fluid property in a non-contact seal, in particular a labyrinth seal (40), between the rotor stage (29) and the stator stage (30), wherein the measurement is carried out by the sensor element (51) by contact with the flow of the fluid along the flow path (S) in the non-contact seal (40) during operation, wherein the sensor element (51) is arranged at the start, in the middle or at the end of the flow path (S) through the non-contact seal, wherein the seal (40) with the sensor element (51) is in the vicinity of the gas path in order to provide sealing against the ingress of hot gas and possibly to detect an ingress of hot gas.

## Revendications

1. Agencement de capteur (50) comprenant un élément de capteur (51) pour la mesure d'au moins une propriété de fluide physique et/ou chimique dans une turbomachine (10) qui présente une turbine (16, 17, 18), en particulier une turbine haute pression (16), dans laquelle sont disposés un étage de rotor (29) et un étage de stator (30),
**caractérisé en ce que**
l'élément de capteur (51) détecte l'au moins une propriété de fluide dans un joint d'étanchéité sans contact, en particulier un joint d'étanchéité à labyrinthe (40), entre l'étage de rotor (29) et l'étage de stator (30), l'élément de capteur (51), pendant le fonctionnement, étant en contact avec l'écoulement de fluide (41) le long du chemin d'écoulement (S) dans le joint d'étanchéité sans contact (40), l'élément de capteur (51) étant disposé au début, au milieu ou à la fin du chemin d'écoulement (S) à travers le joint d'étanchéité sans contact, le joint d'étanchéité (40) avec l'élément de capteur (51) étant à proximité du chemin de gaz afin de réaliser l'étanchéité contre l'entrée de gaz chauds et de détecter éventuellement une entrée de gaz chauds.

2. Agencement de capteur selon la revendication 1, **caractérisé en ce qu'**il existe un gradient de pression sur le chemin d'écoulement (S), de sorte qu'un fluide soit transporté à travers le joint d'étanchéité sans contact, en particulier le joint d'étanchéité à labyrinthe (40).

3. Agencement de capteur selon au moins l'une quelconque des revendications précédentes, **caractérisé en ce que** l'élément de capteur (51) est positionné dans une chambre de mesure qui est connectée par un canal au point de mesure proprement dit dans le joint d'étanchéité (40), et de ce fait, le fluide à détecter doit d'abord être guidé à travers un canal jusqu'à l'élément de capteur (51).

4. Agencement de capteur selon au moins l'une quelconque des revendications précédentes, **caractérisé en ce que** l'élément de capteur (51) permet de mesurer une température de l'écoulement de fluide (41), en particulier un écoulement d'air, un débit quantitatif de l'écoulement de fluide (41), en particulier un débit d'huile et/ou une composition de l'écoulement de fluide (41), en particulier d'un gaz de combustion.

5. Agencement de capteur selon au moins l'une quelconque des revendications précédentes, **caractérisé en ce que** l'élément de capteur (51) est accouplé à un dispositif de commande (52) avec lequel un signal de commande (53) peut être délivré en fonction de la mesure avec l'élément de capteur (51) .

6. Procédé de mesure comprenant un élément de capteur (51) pour la mesure d'au moins une propriété de fluide physique et/ou chimique dans une turbomachine (10) qui présente une turbine (16, 17, 18), en particulier une turbine haute pression (16), dans laquelle sont disposés un étage de rotor (29) et un étage de stator (30),
**caractérisé en ce que**
l'élément de capteur (51) détecte l'au moins une propriété de fluide dans un joint d'étanchéité sans contact, en particulier un joint d'étanchéité à labyrinthe (40), entre l'étage de rotor (29) et l'étage de stator (30), la mesure s'effectuant pendant le fonctionnement par l'élément de capteur (51) par un contact avec l'écoulement du fluide le long du chemin d'écoulement (S) dans le joint d'étanchéité sans contact (40), l'élément de capteur (51) étant disposé au début, au milieu ou à la fin du chemin d'écoulement (S) à travers le joint d'étanchéité sans contact, le joint d'étanchéité (40) avec l'élément de capteur (51) étant à proximité du chemin de gaz afin de réaliser l'étanchéité contre l'entrée de gaz chauds et de détecter éventuellement une entrée de gaz chauds.
